# EUROPÄISCHE PATENTSCHRIFT

(11) **EP 2 872 471 B1**
(45) Veröffentlichungstag und Bekanntmachung des Hinweises auf die Patenterteilung: **29.06.2016**
(21) Anmeldenummer: 13734781.1
(22) Anmeldetag: 09.07.2013
(51) Int. Cl.: C07C 7/10, C07C 5/29

(54) **CHEMISCHES UMSETZUNGSVERFAHREN IN EINER DISPERSION**
CHEMICAL CONVERSION PROCESS IN A DISPERSION
PROCÉDÉ DE MISE EN RÉACTION CHIMIQUE DANS UNE DISPERSION

(30) Priorität: 11.07.2012 EP 12175827
(43) Veröffentlichungstag der Anmeldung: 20.05.2015
(73) Patentinhaber: BASF SE, 67056 Ludwigshafen am Rhein (DE)
(72) Erfinder: TSCHIRSCHWITZ, Steffen, 68199 Mannheim (DE); JONI, Joni, 65843 Sulzbach (DE); BÜRKLE, Jochen, 68163 Mannheim (DE); PFEIFFER, Daniel, 67435 Neustadt (DE); HÜBNER, Michael, 68623 Lampertheim (DE); HOLUB, Nicole, 68167 Mannheim (DE); BITTERLICH, Stefan, 67246 Dirmstein (DE)
(74) Vertreter: BASF IP Association
(86) Internationale Anmeldenummer: PCT/EP2013/064441
(87) Internationale Veröffentlichungsnummer: WO 2014/009341

(56) Entgegenhaltungen:
- EP-A1- 1 310 472
- V. A. KSENOFONTOV ET.AL.: "Isomerization of cyclic hydrocarbons mediated by an AlCl3 - based ionic liquid as catalyst", REACT. KINET. CATAL. LETT., Bd. 80, Nr. 2, 2003, Seiten 329-335, XP002714086,

## Beschreibung

Die vorliegende Erfindung betrifft ein chemisches Umsetzungsverfahren, vorzugsweise ein Isomerisierungsverfahren, von mindestens einem Kohlenwasserstoff in Gegenwart einer ionischen Flüssigkeit. Die chemische Umsetzung wird in einer Dispersion durchgeführt, wobei in der Dispersion der Kohlenwasserstoff (Phase (B)) in der ionischen Flüssigkeit (Phase (A)) dispergiert ist und das Volumenverhältnis der Phase (A) zu Phase (B) im Bereich von 2,5 bis 4 zu 1 [Vol/Vol] liegt.

Ionische Flüssigkeiten, insbesondere saure ionische Flüssigkeiten, eignen sich unter anderem als Katalysatoren für die Isomerisierung von Kohlenwasserstoffen. Eine entsprechende Verwendung einer ionischen Flüssigkeit ist beispielsweise in WO 2011/069929 offenbart, wo eine spezielle Auswahl von ionischen Flüssigkeiten in Gegenwart eines Olefins zur Isomerisierung von gesättigten Kohlenwasserstoffen eingesetzt wird, insbesondere zur Isomerisierung von Methylcyclopentan (MCP) zu Cyclohexan. In dem Verfahren gemäß WO 2011/069929 können die zu isomerisierenden Kohlenwasserstoffe und die ionischen Flüssigkeiten prinzipiell in beliebigen Verhältnissen eingesetzt werden. In den Ausführungsbeispielen wird ein fünffacher Volumenüberschuss an ionischen Flüssigkeiten eingesetzt. Ein sinngemäßes Verfahren ist in WO 2011/069957 beschrieben, allerdings erfolgt dort die Isomerisierung nicht in Gegenwart eines Olefins, sondern mit einer Kupfer (II)-Verbindung.

US-A 2003/0109767 offenbart ein Verfahren zur Isomerisierung von C₅-C₈-Paraffinkohlenwasserstoffen (Paraffinen) in Gegenwart einer ionischen Flüssigkeit als Katalysator. Die ionische Flüssigkeit umfasst als Kationen stickstoffhaltige Heterocyclen oder stickstoffhaltige Aliphate, die entsprechenden Anionen sind von Metallhalogeniden abgeleitet. Bei den zu isomerisierenden Paraffinen handelt es sich um lineare Alkane wie n-Hexan oder n-Octan sowie monosubstituierte Alkane wie 3-Methylhexan bzw. Mischungen davon. Durch das in US-A 2003/0109767 beschriebene Verfahren sollen Paraffine mit einem höheren Verzweigungsgrad hergestellt werden. Hinsichtlich der Mengenverhältnisse von ionischer Flüssigkeit zu zu isomerisierendem Kohlenwasserstoff sind prinzipiell keine Beschränkungen enthalten, in den Ausführungsbeispielen werden Mengen in einem Verhältnis von 1 : 1 oder maximal einem 1,5-fachen Überschuss an ionischer Flüssigkeit eingesetzt.

In dem in EP-A 1 503 236 beschriebenen Isomerisierungsverfahren soll ebenfalls ein höherer Verzweigungsgrad in den in Gegenwart einer ionischer Flüssigkeit zu isomerisierenden Paraffinen (Kohlenwasserstoffen) erhalten werden. Das Isomerisierungsverfahren wird zudem in Gegenwart von zyklischen Kohlenwasserstoffen als Additiven und in einem Reaktionsmedium durchgeführt, wobei die zyklischen Kohlenwasserstoffe ein tertiäres Kohlenstoffatom als Struktureinheit enthalten bzw. durch das Reaktionsmedium in eine entsprechende Verbindung mit einer solchen Struktureinheit überführt werden. Vorzugsweise werden Methylcyclohexan oder Dimethylcyclopentan als solche zyklischen Kohlenwasserstoffadditive eingesetzt. Als zu isomerisierende Paraffine werden lineare Alkane wie n-Butan oder n-Octan sowie monomethylsubstituierte Alkane wie 2-Methylhexan eingesetzt. Die ionischen Flüssigkeiten basieren vorzugsweise auf stickstoffhaltigen Heterocyclen oder stickstoffhaltigen Aliphaten als Kationen sowie auf anorganischen Anionen wie Aluminiumhalogeniden. In EP-A 5 403 236 sind keine konkreten Angaben enthalten, in welchem Verhältnis die als Katalysator verwendete ionische Flüssigkeit gegenüber den zu isomerisierenden Paraffinen eingesetzt wird.

V.A. Ksenofontov et.a., React. Kinet. Catal. Lett. Vol. 80, No. 2, 329-335 (2003) offenbart ein Verfahren zu Isomerisierung von Cyclohexan zu Methylcyclopentan.

Im Allgemeinen sind ionische Flüssigkeiten einerseits und Kohlenwasserstoffe (organische Phasen) andererseits nicht oder nur sehr schwer mischbar, sie bilden zwei getrennte Phasen aus. Um die genannte Katalysewirkung nutzen zu können, muss ein intensiver Kontakt zwischen organischer Phase und der ionischer Flüssigkeit hergestellt werden. Hierzu werden die beiden Phasen häufig in Rührkesseln mit intensivem Rühren unter Erhalt von Dispersionen durchmischt. In Abhängigkeit von Parametern wie Art der ionischen Flüssigkeit bzw. der organischen Phase oder dem Phasenverhältnis kann die Dispersion entweder als Dispersion einer ionischen Flüssigkeit in der organischen Phase vorliegen oder es kann sich um eine Dispersion der organischen Phase in der ionischen Flüssigkeit handeln. Unabhängig von der konkret vorliegenden Dispergierrichtung ist es bei solchen Dispersionen ein generelles Problem, die dispergierte Phase im Anschluss an die Reaktion von der zusammenhängenden Phase abzutrennen.

WO 2010/062922 offenbart ein mehrstufiges Verfahren zur Trennung einer ionischen Flüssigkeit von Kohlenwasserstoffen unter Verwendung eines Koaleszierfilters. Das Koaleszierfiltermaterial muss so beschaffen sein, dass es eine stärkere Affinität für die ionische Flüssigkeit als für die Kohlenwasserstoffe hat. Als Koaleszierfiltermaterialien eignen sich gemäß WO 2010/062922 Glaskugeln, Edelstahl, Glasfasern, Polymerfasern oder organische Membrane, insbesondere Glasfasern. Im Koaleszierfilter wird eine Trennung der ionischen Flüssigkeit aus den Kohlenwasserstoffen bewirkt.

Die der vorliegenden Erfindung zugrunde liegende Aufgabe besteht in der Bereitstellung eines neuen Verfahrens zur chemischen Umsetzung von mindestens einem Kohlenwasserstoff in Gegenwart einer ionischen Flüssigkeit, insbesondere zu Isomerisierung von mindestens einem Kohlenwasserstoff in Gegenwart einer ionischen Flüssigkeit.

Gelöst wird die Aufgabe durch ein chemisches Umsetzungsverfahren von mindestens einem Kohlenwasserstoff in Gegenwart einer ionischen Flüssigkeit, dadurch gekennzeichnet, dass die chemische Umsetzung in einer Dispersion (D1) durchgeführt wird, wobei in der Dispersion (D1) die Phase (B) in der Phase (A) dispergiert ist, das Volumenverhältnis der Phase (A) zu Phase (B) im Bereich von 2,5 bis 4 zu 1 [Vol/Vol] liegt, die Phase (A) zu größer 50 Gew.-% mindestens eine ionische Flüssigkeit enthält und die Phase (B) zu größer 50 Gew.-% mindestens einen Kohlenwasserstoff enthält..

Die vorliegende Anmeldung betrifft ein Verfahren zur Isomerisierung von Methylcyclopentan (MCP) zu Cyclohexan in Gegenwart einer ionischen Flüssigkeit, dadurch gekennzeichnet, dass die Isomerisierung von Methylcyclopentan (MCP) zu Cyclohexan in einer Dispersion (D1) durchgeführt wird, wobei in der Dispersion (D1) die Phase (B) in der Phase (A) dispergiert ist, das Volumenverhältnis der Phase (A) zu Phase (B) im Bereich von 2,5 bis 4 zu 1 [Vol/Vol] liegt, die Phase (A) zu größer 50 Gew.-% mindestens eine ionische Flüssigkeit enthält und die Phase (B) zu größer 50 Gew.-% mindestens einen Kohlenwasserstoff enthält.

Durch das erfindungsgemäße Verfahren kann in vorteilhafter Weise eine chemische Umsetzung, insbesondere eine Isomerisierung, von Kohlenwasserstoffen durchgeführt werden. Aufgrund des speziellen Volumenverhältnisses der Phase (A) zu Phase (B) im Bereich von 2,5 bis 4 zu 1 [Vol/Vol] kann eine höhere Raum-Zeit-Ausbeute erzielt werden. Aufgrund dieser Optimierung kann auch der apparative Aufwand zur Durchführung des Verfahrens verringert werden, beispielsweise kann die Vorrichtung, in der die chemische Umsetzung, insbesondere die Isomerisierung, durchgeführt wird, klein gehalten werden. Es können also kleinere Reaktoren, beispielsweise im Fall einer Isomerisierung die Dimensionierung und/oder die Anzahl der eingesetzten Rührkessel, eingesetzt werden.

Sofern im Rahmen der vorliegenden Erfindung im Anschluss an die chemische Umsetzung mit der Dispersion (D1) ein Kippen der Dispergierrichtung durchgeführt wird (d.h. Umkehrung der Dispergierrichtung so dass nach dem Kippen die Phase (A) in der Phase (B) dispergiert ist), kann ein zusätzlicher Vorteil hinsichtlich des Abtrennens der ionischen Flüssigkeit von den Kohlenwasserstoffen erzielt werden, weil dann die Phasentrennung schneller abläuft als im umgekehrten Fall, was eine Reduzierung des apparativen Aufwandes für die Phasentrennung erlaubt.

Der Begriff "Kippen der Dispergierrichtung" wird im nachfolgenden Text im Zusammenhang mit der Einleitung des Stromes (S2), der einen Überschuss an Phase (B) enthält, in den Strom (S1) im Rahmen einer Ausführungsform des erfindungsgemäßen Verfahrens näher definiert.

Sofern im Rahmen der vorliegenden Erfindung im Anschluss an das Kippen der Dispergierrichtung und den damit verbundenen Abtrennungsschritt in einer Phasentrenneinheit, vorzugsweise in einem Phasenscheider, ein weiterer Trennschritt unter Verwendung eines Koaleszierfilters, insbesondere eines Koaleszierfilters aus Acrylphenolharz oder Glasfaser, oder unter Verwendung eines ein Gestrick enthaltenden Nachabscheiders durchgeführt wird, wird zusätzlich eine verbesserte Abtrennung von in feindisperser Form und/oder in kleinen Mengen vorliegenden höherviskosen Komponenten, insbesondere ionische Flüssigkeiten, festgestellt. Nachfolgend wird das erfindungsgemäße chemische Umsetzungsverfahren, das in einer Dispersion durchgeführt wird, von mindestens einem Kohlenwasserstoff in Gegenwart einer ionischen Flüssigkeit näher definiert.

Unter dem Begriff "chemisches Umsetzungsverfahren" oder "chemische Umsetzung" wird im Rahmen der vorliegenden Erfindung prinzipiell jede dem Fachmann bekannte chemische Umsetzung oder chemische Reaktion verstanden, bei der mindestens ein Kohlenwasserstoff chemisch umgesetzt, modifiziert oder in einer sonstigen Weise hinsichtlich seiner Zusammensetzung oder Struktur verändert wird.

Bevorzugt ist das chemische Umsetzungsverfahren ausgewählt aus einer Alkylierung, einer Polymerisation, einer Dimerisierung, einer Oligomerisierung, einer Acylierung, einer Metathese, einer Polymerisation oder Copolymerisation, einer Isomerisierung, einer Carbonylierung oder Kombinationen davon. Alkylierungen, Isomerisierungen, Polymerisationen etc. sind dem Fachmann bekannt. Besonders bevorzugt im Rahmen der vorliegenden Erfindung ist das chemische Umsetzungsverfahren eine Isomerisierung.

Im Rahmen der vorliegenden Erfindung enthält die Phase (A) mindestens eine ionische Flüssigkeit, wobei der Anteil an ionischer Flüssigkeit in der Phase (A) größer als 50 Gew.-% ist (bezogen auf die Summe aller Komponenten der Phase (A)). Die Phase (A) ist vorzugsweise eine ionische Flüssigkeiten enthaltende Phase, die nicht oder nur sehr schwer mit Kohlenwasserstoffen mischbar ist und/oder die maximal 10 Gew.-% an Kohlenwasserstoffen enthält.

Beispielsweise können in der Phase (A) Gemische aus zwei oder mehr ionischen Flüssigkeiten enthalten sein, vorzugsweise enthält die Phase (A) eine ionische Flüssigkeit. Neben der ionischen Flüssigkeit können in der Phase (A) auch weitere Komponenten enthalten sein, die mit der ionischen Flüssigkeit mischbar sind. Bei solchen Komponenten kann es sich beispielsweise um Cokatalysatoren handeln, die bei Isomerisierungsreaktionen unter Verwendung von ionischen Flüssigkeiten eingesetzt werden. Ein bevorzugtes Beispiel für solche Cokatalysatoren sind Halogenwasserstoffe, insbesondere Chlorwasserstoff. Darüber hinaus können in der Phase (A) auch Bestandteile oder Zerfallsprodukte der ionischen Flüssigkeiten, die beispielsweise während des Isomerisierungsprozesses entstehen können, enthalten sein, wie Aluminiumchlorid. Vorzugsweise ist bei der Phase (A) der Anteil an ionischer Flüssigkeit größer 80 Gew.-% (bezogen auf die Summe aller Komponenten der Phase (A)).
Als ionische Flüssigkeiten eignen sich im Rahmen der vorliegenden Erfindung prinzipiell alle dem Fachmann bekannten ionischen Flüssigkeiten, sofern sie die jeweils durchgeführte Reaktion selbst katalysieren oder ein Lösungsvermögen für den jeweils eingesetzten Katalysator besitzen.. Ein Überblick hinsichtlich geeigneter ionischer Flüssigkeiten kann für den Fall der Isomerisierung beispielsweise WO 2011/069929 entnommen werden. Bevorzugt ist im Rahmen der vorliegenden Erfindung eine saure ionische Flüssigkeit. Vorzugsweise ist die in der Phase (A) enthaltene ionische Flüssigkeit eine ionische Flüssigkeit, insbesondere eine saure ionische Flüssigkeit, mit der Zusammensetzung K1AlₙX₍₃ₙ₊₁₎, wobei K1 ein einwertiges Kation, X gleich Halogen und 1 < n < 2,5, ist. K1 ist vorzugsweise ein unsubstituiertes oder zumindest teilweise alkyliertes Ammoniumion oder ein heterocyclisches (einwertiges) Kation, insbesondere ein Pyridiniumion, ein Imidazoliumion, ein Pyridaziniumion, ein Pyrazoliumion, ein Imidazoliniumion, ein Thiazoliumion, ein Triazoliumion, ein Pyrrolidiniumion, ein Imidazolidiniumion oder ein Phosphoniumion. X ist vorzugsweise Chlor oder Brom.

Mehr bevorzugt enthält die ionische Flüssigkeit, insbesondere die saure ionische Flüssigkeit, als Kation ein zumindest teilweise alkyliertes Ammoniumion oder ein heterocyclisches Kation und/oder als Anion ein Chloroalumination mit der Zusammensetzung AlₙCl₍₃ₙ₊₁₎ mit 1 < n < 2,5. Vorzugsweise enthält das zumindest teilweise alkylierte Ammoniumion einen, zwei oder drei Alkylreste mit (jeweils) 1 bis 10 Kohlenstoffatomen. Sofern zwei oder drei Alkylsubstituenten mit den entsprechenden Ammoniumionen vorhanden sind, kann die jeweilige Kettenlänge unabhängig voneinander gewählt werden, vorzugsweise weisen alle Alkylsubstituenten die gleiche Kettenlänge auf. Besonders bevorzugt sind trialkylierte Ammoniumionen mit einer Kettenlänge von 1 bis 3 Kohlenstoffatomen. Das heterocyclische Kation ist vorzugsweise ein Imidazoliumion oder ein Pyridiniumion.

Besonders bevorzugt enthält die ionische Flüssigkeit, insbesondere die saure ionische Flüssigkeit, als Kation ein zumindest teilweise alkyliertes Ammoniumion und als Anion ein Chloroalumination mit der Zusammensetzung AlₙCl₍₃ₙ₊₁₎ mit 1 < n < 2,5. Beispiele für solche besonders bevorzugten ionischen Flüssigkeiten sind Trimethylammoniumchloroaluminat und Triethylammoniumchloroaluminat.

Die Phase (B) ist im Rahmen der vorliegenden Erfindung dadurch charakterisiert, dass sie mindestens einen Kohlenwasserstoff enthält, wobei der Gehalt an Kohlenwasserstoff in der Phase (B) größer als 50 Gew.-% ist (bezogen auf die Summe aller Komponenten der Phase (B)). Die Phase (B) ist vorzugsweise eine kohlenwasserstoffhaltige Phase, die nicht oder nur sehr schwer mit ionischen Flüssigkeiten mischbar ist und/oder die maximal 1 Gew.-% an ionischen Flüssigkeiten (bezogen auf das Gesamtgewicht der Phase) enthält.

Die konkrete Zusammensetzung der Phase (B) ist abhängig vom gewählten chemischen Umsetzungsverfahren. Die Phase (B) hat zu Beginn eines chemischen Umsetzungsverfahrens eine andere Zusammensetzung als nach Beendigung des entsprechenden chemischen Umsetzungsverfahrens. In anderen Worten ausgedrückt bedeutet dies, dass die Phase (B) ihre Zusammensetzung aufgrund der Durchführung des jeweiligen chemischen Umsetzungsverfahrens ändert. Prinzipiell können in der Phase (B) beliebige Kohlenwasserstoffe enthalten sein. Der Fachmann weiß aufgrund seines allgemeinen Fachwissens, für welches konkrete chemische Umsetzungsverfahren welche Kohlenwasserstoffe und in welchen Zusammensetzungen besten geeignet sind. Gegebenenfalls können in der Phase (B) auch Verbindungen enthalten sein, die selber keine Kohlenwasserstoffe, jedoch mit diesen mischbar sind. Im nachfolgenden Text wird die Zusammensetzung der Phase (B) anhand der im Rahmen der vorliegenden Erfindung als chemischer Umsetzung bevorzugten Isomerisierung verdeutlicht.

Vorzugsweise enthält die Phase (B) vor der chemischen Umsetzung, insbesondere vor der Isomerisierung, als Kohlenwasserstoff Methylcyclopentan (MCP) oder ein Gemisch aus Methylcyclopentan (MCP) mit mindestens einem weiteren Kohlenwasserstoff, ausgewählt aus Cyclohexan, n-Hexan, iso-Hexane n-Heptan, iso-Heptane oder Dimethylcyclopentane.

Mehr bevorzugt enthält die Phase (B) vor der chemischen Umsetzung, insbesondere vor der Isomerisierung, ein Gemisch aus Methylcyclopentan (MCP) mit mindestens einem weiteren Kohlenwasserstoff, ausgewählt aus Cyclohexan, n-Hexan, iso-Hexane n-Heptan, iso-Heptane oder Dimethylcyclopentane, wobei das Konzentrationsverhältnis MCP/Cyclohexan vorzugsweise mindestens 0,2 beträgt

Besonders bevorzugt wird im Rahmen der vorliegenden Erfindung Methylcyclopentan (MCP) zu Cyclohexan isomerisiert.

Vorzugsweise enthält die Phase (B) nach der chemischen Umsetzung, insbesondere nach der Isomerisierung, als Kohlenwasserstoff Cyclohexan oder ein Gemisch aus Cyclohexan mit mindestens einem weiteren Kohlenwasserstoff, ausgewählt aus Methylcyclopentan (MCP), n-Hexan, iso-Hexan, n-Heptan, iso-Heptan oder Dimethylcyclopentan.

Besonders bevorzugt enthält die Phase (B) nach der chemischen Umsetzung, insbesondere nach der Isomerisierung, ein Gemisch aus Cyclohexan, MCP und mindestens einem weiteren Kohlenwasserstoff. Vorzugsweise ist der weitere Kohlenwasserstoff ausgewählt aus n-Hexan, iso-Hexan, n-Heptan, iso-Heptan oder Dimethylcyclopentan. Insbesondere ist der Anteil an verzweigten Kohlenwasserstoffen im Gemisch (nach der Isomerisierung) kleiner 5 Gew.-% (bezogen auf die Summe aller Komponenten der Phase (B)). Weiterhin ist es im erfindungsgemäßen Verfahren bevorzugt, dass nach der Isomerisierung im erhaltenen Gemisch, das in der Phase (B) enthalten ist, ein geringerer Anteil an MCP und offenkettigen linearen Kohlenwasserstoffen vorliegt im Vergleich zu der entsprechenden Zusammensetzung der Phase (B) vor der Isomerisierung.

Im Rahmen der vorliegenden Erfindung erfolgt die chemischen Umsetzung, insbesondere die Isomerisierung, in einer Dispersion (D1), in der die Phase (B) in der Phase (A) dispergiert ist. Die Dispergierrichtung (d.h. die Information, welche Phase in disperser Form in der der jeweils anderen Phase vorliegt) kann bestimmt werden, indem eine Probe, ggf. nach Zusatz eines selektiv die eine Phase einfärbenden Farbstoffs, unter einem Lichtmikroskop mit Durchlicht untersucht wird. Die Phasen (A) und (B) weisen dabei die vorstehenden Definitionen auf.

Die Dispersion (D1) kann nach dem Fachmann bekannten Methoden hergestellt werden, beispielsweise kann eine solche Dispersion durch intensives Verrühren der Phasen erzeugt werden. In der Dispersion (D1) liegt das Volumenverhältnis der Phase (A) zu Phase (B) im Bereich von 2,5 bis 4 zu 1 [Vol/Vol], bevorzugt im Bereich von 2,5 bis 3 zu 1 [Vol/Vol].

Im nachfolgenden Text werden weitere und/oder bevorzugte Ausführungsformen der vorliegenden Erfindung aufgeführt, die gegebenenfalls anhand der für ein chemisches Umsetzungsverfahren bevorzugten Isomerisierung verdeutlicht werden. Sämtliche nachfolgenden Ausführungsformen der vorliegenden Erfindung können auch für andere chemische Umsetzungsverfahren (als eine Isomerisierung) eingesetzt werden.

Vorzugsweise wird die Isomerisierung (chemische Umsetzung) in einem Reaktionsapparat oder einer Kaskade von Reaktionsapparaten durchgeführt, insbesondere erfolgt dies in einem Rührkessel oder einer Rührkesselkaskade.

Weiterhin ist es bevorzugt, dass die Dispersion (D1) zusätzlich einen Halogenwasserstoff enthält und/oder ein gasförmiger Halogenwasserstoff in Kontakt mit der Dispersion (D1) gebracht wird, vorzugsweise ist der Halogenwasserstoff HCl. Besonders bevorzugt enthält D1 zusätzlich HCl und/oder gasförmiges HCl wird in die Dispersion (D1) eingeleitet.

Weiterhin kann aus der Vorrichtung, in der die chemische Umsetzung durchgeführt wird, ein Strom (S1) ausgeleitet werden, wobei der Strom (S1) zumindest eine Teilmenge der Dispersion (D1), in der die Phase (B) in der Phase (A) dispergiert ist, enthält und in der Phase (B) mindestens ein Kohlenwasserstoff enthalten ist, der bei der chemischen Umsetzung hergestellt wurde. Der Vollständigkeit halber wird darauf hingewiesen, dass im Strom (S1) die Dispersion (D1) die gleiche chemische Zusammensetzung (inklusive der Mengenverhältnisse) hat wie in der Vorrichtung, in der die chemische Umsetzung durchgeführt wird, sofern die chemische Umsetzung kontinuierlich durchgeführt wird. Der Strom (S1) wiederum wird vorzugsweise in eine Phasentrenneinheit eingeleitet. Phasentrenneinheiten als solche sind dem Fachmann bekannt. Bei dieser Phasentrenneinheit handelt es sich vorzugsweise um einen Phasenscheider.

In der Phasentrenneinheit kann vorzugsweise ein Strom (S4), enthaltend mindestens 70 Gew.-%, bevorzugt mindestens 90 Gew.-%, an Phase (A), und ein Strom (S5), enthaltend mindestens 70 Gew.-%, bevorzugt mindestens 90 Gew.-%,an Phase (B), voneinander abgetrennt werden. Weiterhin ist es bevorzugt, dass der Strom (S4) in die Vorrichtung, in der die chemische Umsetzung, vorzugsweise die Isomerisierung, durchgeführt wird, rückgeleitet wird. Die vorstehenden Angaben in Gew.-% beziehen sich auf die entsprechenden Mengen, die im Strom (S1) enthalten sind.

In einer weiteren bevorzugten Ausführungsform wird in den Strom (S1) ein Strom (S2), enthaltend mindestens 70 Gew.-%, bevorzugt mindestens 90 Gew.-%, an Phase (B), eingeleitet. Die vorstehenden Angaben in Gew.-% beziehen sich auf das Gesamtgewicht des Stroms (S2). Dabei wird ein Strom (S3) ausgebildet, enthaltend eine Dispersion (D2), in der die Phase (A) in der Phase (B) dispergiert ist, d.h. es kommt zum Kippen der Dispergierrichtung. Dieser Strom (S3) wiederum wird anstelle des Stroms (S1) in die vorstehend beschriebene Phasentrenneinheit eingeleitet. Gegebenenfalls kann der Strom (S2) auch direkt in die Phasentrenneinheit oder an eine sonstige Stelle im Verfahren rückgeleitet werden. Vorzugsweise erfolgt das Einleiten des Stroms (S2) in den Strom (S1) in einem Rührbehälter oder statischen Mischer, in dem der Strom (S3) ausgebildet wird.

Weiterhin ist es bevorzugt, dass der Strom (S2) aus dem aus der Phasentrenneinheit erhaltenen Strom (S5) abgezweigt und gegebenenfalls in den Strom (S1) vor oder in die Phasentrenneinheit rückgeleitet wird.

In der Regel werden zwischen 50 und 90 % der Gesamtmenge des Stroms (S5) als Strom (S2) abgetrennt und in den Strom (S1) rückgeführt. Allerdings ist es auch denkbar, dass zumindest temporär größere Mengen oder der Strom (S5) sogar vollständig rückgeleitet werden

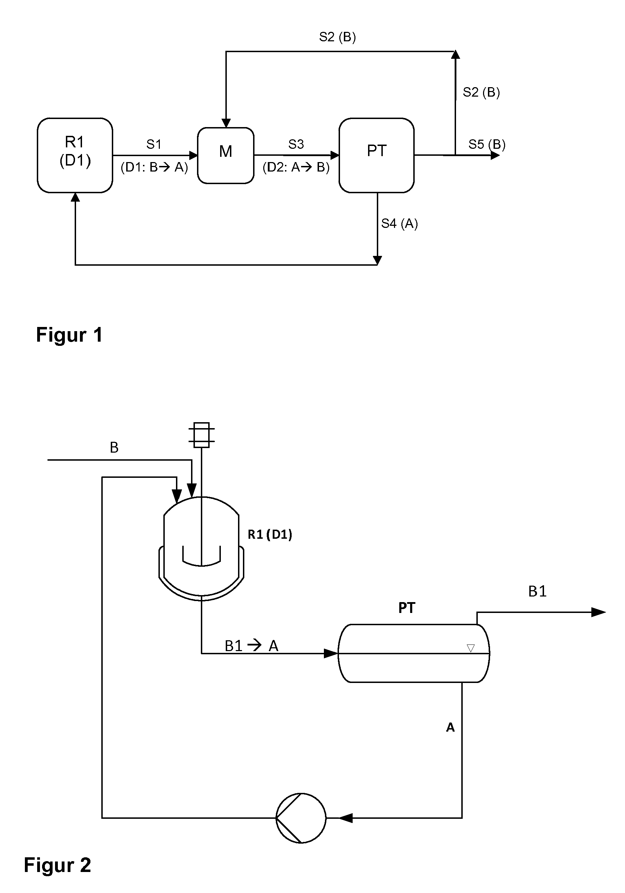

In Figur 1 wird das erfindungsgemäße Verfahren gemäß einer bevorzugten Ausführungsform nochmals verdeutlicht. In dieser Ausführungsform, die vorzugsweise als Isomerisierung durchgeführt wird, erfolgt die chemische Umsetzung in Reaktionsapparaten oder eine Kaskade von Reaktionsapparaten (R1) wie z.B. Rührkesseln. Aus (R1) wird der Strom (S1), enthaltend die Dispersion (D1), in einer Mischvorrichtung (M) mit dem zurück geleiteten Strom (S2) unter Ausbildung der Dispersion (D2) vereinigt. Der Strom (S3) wird wiederum in eine Phasentrenneinheit (PT), insbesondere in einen Phasenscheider, eingeleitet. Zum besseren Verständnis sind in Figur 1 unter den jeweiligen Strömen in Klammern die darin enthaltenen Hauptkomponenten angegeben. Bei den Strömen (S1) und (S3) ist im jeweiligen Klammerausdruck auch die Dispergierrichtung der jeweiligen Dispersionen mit berücksichtigt, wobei der Pfeil die Dispergierrichtung zum Ausdruck bringt. Dies bedeutet, dass beispielsweise die im Strom (S1) enthaltene Dispersion (D1) eine Phase (B) aufweist, die in der Phase (A) dispergiert ist.

In einer weiteren bevorzugten Ausführungsform der vorliegenden Erfindung wird mit dem Strom (S5), der wie vorstehend beschrieben in einer Phasentrenneinheit anfällt, (mindestens) ein weiterer Phasentrennungsschritt durchgeführt. Dieser weitere Phasentrennungsschritt wird vorzugsweise durchgeführt, um im Strom (S5) enthaltene Restmengen an Phase (A) abzutrennen. Besonders bevorzugt ist im Strom (S5) nach Durchführung dieses weiteren Phasentrennungsschritt keine oder nur eine geringe Menge an Phase (A) enthalten (< 50 Gew.-ppm). Dieser weitere Phasentrennungsschritt kann direkt im Anschluss an den ersten Phasentrennungsschritt erfolgen oder erst im Anschluss an die Abtrennung des Stroms (S2) gemäß der vorstehend beschriebenen Variante. Vorzugsweise erfolgt dieser weitere Phasentrennungsschritt im Anschluss an die Abtrennung des Stroms (S2).

Vorrichtungen zur Durchführung dieses weiteren Phasentrennungsschritts, im Folgenden "Nachabscheider" genannt, sind dem Fachmann bekannt. Vorzugsweise werden hierfür Vorrichtungen verwendet, die geeignet sind, Restmengen oder kleinere Mengen (< 2,5 Gew.-% bezogen auf die zu trennende Gesamtmenge) abzutrennen. Bevorzugte Vorrichtungen sind Koaleszierfilter oder ein nachfolgender Phasenscheider mit oder ohne Einbauten. Mögliche Einbauten sind Gestricke, Füllkörper, Packungen oder Röhren.

Vorzugsweise wird dies so durchgeführt, dass nach Abtrennung des Stroms (S2) der Strom (S5) durch den im vorhergehenden Absatz beschriebenen Nachabscheider geleitet wird, um im Strom (S5) verbliebene Restmengen an Phase (A) abzutrennen. Besonders bevorzugt ist im Strom (S5) nach Durchführung dieses weiteren Phasentrennungsschritt keine oder nur eine geringe Menge an Phase (A) enthalten (< 50 Gew.-ppm).

Sofern ein Koaleszierfilter verwendet wird, handelt es sich vorzugsweise um einen Koaleszierfilter aus Glasfaser oder Acrylphenolharz, insbesodere aus Acrylphenolharz. Koaleszierfilter aus Acrylphenolharz sind beispielsweise kommerziell erhältlich von der Firma Fuhr GmbH (Deutschland) oder von dem Hersteller CUNO Fluid Purification. Solche geeigneten Koaleszierfilter (K) haben Feinheiten von 1-150 µm, bevorzugt 10, 25 oder 50 µm, besonders bevorzugt 10 µm. Weiterhin sind 2 Versionen bezüglich der Oberfläche möglich: gerillt und ungerillt, bevorzugt ist ungerillt. Die Kerzen des Koaleszierfilter (K) als solche haben beispielsweise einen Innendurchmesser von 27 mm und einen Außendurchmesser von 65 mm und sind in Längen von 4" bis 60" erhältlich. Die Kerze ist vorzugsweise eine asymmetrische, harzgebundene und stützkernlose Filterkerze. Vorzugsweise enthält sie im Wesentlichen Acrylfasern, die mit Phenolharz gebunden werden.

Der Koaleszierfilter kann in eine größere Einheit, beispielsweise einen Filterbehälter, integriert sein. Vorzugsweise wird im Rahmen der vorliegenden Erfindung unter einem Koaleszierfilter, der aus Glasfaser oder Acrylphenolharz hergestellt ist, das Filtermaterial als solches verstanden. Die sonstigen Komponenten der Filtereinheit, beispielsweise der Behälter der Einheit (Filterbehälter) oder das Filtermodul, in dem das Filtermaterial eingebracht ist, können aus anderen Materialien als Glasfaser und/oder Acrylphenolharz hergestellt sein. Der Begriff "hergestellt aus" bedeutet im Rahmen der vorliegenden Erfindung, dass das zur Herstellung des Filtermaterials verwendete Material Glasfaser oder Acrylphenolharz enthält. Vorzugsweise enthält das Filtermaterial mindestens 50 Gew.-%, mehr bevorzugt mindestens 75 Gew.-% und insbesondere mindestens 95 Gew.-% Glasfaser oder Acrylphenolharz

Sofern ein anderer Nachabscheider (auch als Nachphasenschneider bezeichnet) als ein Koaleszierfilter verwendet wird, enthält der Nachabscheider vorzugsweise ein Gestrick, insbesondere ein Glasfasergestrick. Geeignete Gestricke, insbesondere Glasfasergestricke, sind dem Fachmann bekannt, sie sind beispielsweise kommerziell erhältlich von der Firma Rhodius (Deutschland). Bei den bevorzugten Glasfasergestricken handelt es sich um Glasstapelfasern mit einem Faserdurchmesser zwischen 0,1 bis 0,6 mm, bevorzugt zwischen 0,14 bis 0,3 mm. Das Gestrick enthält im Wesentlichen aufgewickelten (Glasstapel-)Fasermatten mit einer Packungsdichte zwischen 100 bis 800 kg/m³, bevorzugt 150 bis 500 kg/m³, besonders bevorzugt 200 bis 400 kg/m³.

Gegebenenfalls kann die aus der Vorrichtung zur Durchführung des weiteren Phasentrennungsschritts abgetrennte Menge an Phase (A) in das erfindungsgemäße Verfahren rückgeleitet werden. Vorzugsweise erfolgt eine solche Rückleitung in den Reaktionsapparat oder die Kaskade von Reaktionsapparaten, in denen die chemische Umsetzung, vorzugsweise eine Isomerisierung, in Gegenwart einer ionischen Flüssigkeit durchgeführt wird. Vorzugsweise wird diese Rückleitung mit dem Strom (S4) vereinigt, der in der ersten Phasentrenneinheit anfällt. Gegebenenfalls können diese die Phase (A) enthaltenen Ströme auch an eine andere Stelle des erfindungsgemäßen Verfahrens rückgeleitet werden, beispielswiese in eine Misch-oder Rührvorrichtung, um die Konzentration der Phase (A) in der Dispersion (D2) zu steuern. Außerdem ist es möglich, dass ein Nachabscheider ohne eine vorgeschaltete "erste Phasentrenneinheit" verwendet wird.

Außerdem ist es möglich, dass aus dem Strom (S5), nachdem in der Vorrichtung zur Durchführung des weiteren Phasentrennungsschritts die Restmenge an Phase (A) abgetrennt wurde, eine weitere Teilmenge abgetrennt wird und diese gegebenenfalls mit dem Strom (S2) vereinigt und in den Strom (S1) rückgeleitet wird.

Aus dem Strom (S5) wird im Rahmen der vorliegenden Erfindung vorzugsweise Cyclohexan isoliert. Verfahren und Vorrichtungen zur Abtrennung von Cyclohexan aus dem Strom (S5), insbesondere wenn es sich um ein Kohlenwasserstoffgemisch handelt, sind dem Fachmann bekannt. Gegebenenfalls können vor der Abtrennung des Cyclohexans noch weitere Aufreinigungsschritte (beispielsweise eine Wäsche mit einer wässrigen und/oder alkalischen Phase) durchgeführt werden, die dem Fachmann bekannt sind.

Nachfolgend wird die Erfindung anhand von Beispielen verdeutlicht.

Für die Versuche werden folgende Substanzen bzw. Zusammensetzungen verwendet:
Ionische Flüssigkeit (A) mit der Zusammensetzung (CH₃)₃NH AlₙCl₃ₙ₊₁ mit n=1,82 laut Elementaranalyse.
Kohlenwasserstoff-Gemisch (B) mit der Zusammensetzung
   - Methylcyclopentan 20 Gew.-%
   - Cyclohexan 50 Gew.-%
   - Hexan 28 %
   - Isohexane (technische Mischung) 2 Gew.-%

Die in den nachfolgenden konkreten Beispielen verwendete Versuchsanordnung ist in Figur 2 schematisch wiedergegeben. Dabei gilt generell Folgendes:
Das Kohlenwasserstoff-Gemisch B (auch als Phase (B) bzw. Organik bezeichnet) wird in einen Rührkessel gegeben, in welchem eine definierte Menge an ionischer Flüssigkeit vorliegt. In diesem findet die Umsetzung des Kohlenwasserstoff-Gemisches, eine Isomerisierung von Methycyclopentan zu Cyclohexan , statt. Der Füllstand des Reaktors (R1) wird hierbei durch Einstellen des variablen Überlaufs zwischen R1 und PT geregelt. Die Dispersion von A und B wird in einen Phasenscheider (PT) geleitet, in welchem sich die beiden Phasen trennen. Die ionische Flüssigkeit als schwerere Phase fällt dabei als untere Phase an und wird durch eine Pumpe wieder zurück in den Behälter R1 befördert. Die obere organische Phase wird abgezogen und per Gaschromatographie auf ihre Zusammensetzung analysiert. Zusätzlich wird mit gasförmiger HCl ein Überdruck von 2 bar im System eingestellt.

### Beispiel 1 (erfinderisches Volumenverhältnis):

Der Füllstand des Reaktors wird auf 450 ml eingestellt. Die Menge an ionischer Flüssigkeit im System wird so gewählt, dass ein Hold-up an IL im Reaktor von 360 ml vorliegt. Daraus ergibt sich ein Organik-Volumen von 90 ml. Die Dispersion (D1), in welcher nun die Phase (B) in der Phase (A) dispergiert ist, besitzt nun ein Volumenverhältnis von 4 zu 1 (Phase (A) zu Phase (B)).

Der Zulauf des Kohlenwasserstoff-Gemisches wird auf 150 g/h gestellt, bei einer Reaktortemperatur von 50°C. Nach 92 Stunden Laufzeit wird der Austrag B1 (150 g/h) mittels Gaschromatographie analysiert. Die Zusammensetzung der Phase B vor der Isomerisierung und B1 danach befindet sich in Tabelle 1.

**Tabelle 1: Zusammensetzung der Phase B vor der Reaktion und der Phase B1 nach der Reaktion.**

| | **Methylcyclopentan** | **Cyclohexan** | **Hexan** | **Isohexane** |
|---|---|---|---|---|
| | **[wt. %]** | **[wt. %]** | **[wt. %]** | **[wt. %]** |
| **Eduktmessung B** | 20,08 | 50,31 | 27,91 | 1,7 |
| **Produktmessung B1** | 12,63 | 57,8 | 22,85 | 6,72 |

Aus diesen Messergebnissen und den Versuchsparametern erhält man folgende Auswertung:

**Tabelle 2: Entsprechende Verweilzeit, Umsätze, Selektivität bezogen auf Cyclohexan und die berechnete Geschwindigkeitskonstante der Isomerisierung.**

| | **Verweilzeit [h]** | **MCP Umsatz [%]** | **Hexan Umsatz [%]** | **Selektivität [CH]** | **k [1/h]** |
|---|---|---|---|---|---|
| **V(A)/V(B)** = **4/1** | 1,661 | 36,7 | 18,13 | 100 | 4,18 |

Die Verweilzeit k berechnet sich hierbei als Quotient aus IL Volumen durch den Organik-Zulauf. Die Konstante k ist hierbei die berechnete Geschwindigkeitskonstante der Isomerisierung von Methycyclopentan zu Cyclohexan für einen Reaktor mit idealer Rückvermischung inklusive Rückreaktion.

### Vergleichsbeispiel 2 (niedrigeres Volumenverhältnis):

Der Füllstand des Reaktors wird auf 450 ml eingestellt. Die Menge an ionischer Flüssigkeit im System wird so gewählt, dass ein Hold-up an IL im Reaktor von 225 ml vorliegt. Daraus ergibt sich ein Organik-Volumen von 225 ml. Die Dispersion (D1) besitzt nun ein Volumenverhältnis von 1 zu 1 (Phase (A) zu Phase (B)).

Der Zulauf des Kohlenwasserstoff-Gemisches wird auf 150 g/h gestellt, bei einer Reaktortemperatur von 50°C. Nach 92 Stunden Laufzeit wird der Austrag B1 (150 g/h) mittels Gaschromatographie analysiert.

**Tabelle 3: Zusammensetzung der Phase B vor der Reaktion und der Phase B1 nach der Reaktion.**

| | **Methylcyclopentan** | **Cyclohexan** | **Hexan** | **Isohexane** |
|---|---|---|---|---|
| | **[wt. %]** | **[wt. %]** | **[wt. %]** | **[wt. %]** |
| **Eduktmessung B** | 20,25 | 50,31 | 27,74 | 1,7 |
| **Produktmessung B1** | 13,76 | 56,80 | 24,49 | 4,95 |

Aus diesen Messergebnissen und den Versuchsparametern erhält man folgende Auswertung:

**Tabelle 4: Entsprechende Verweilzeit, Umsätze, Selektivität bezogen auf Cyclohexan und die berechnete Geschwindigkeitskonstante der Isomerisierung.**

| | **Verweilzeit** [**h**] | **MCP Umsatz [%]** | **Hexan Umsatz [%]** | **Selektivität [CH]** | **k [1/h]** |
|---|---|---|---|---|---|
| **V(A)/V(B)** = **1/1** | 1,038 | 32,05 | 11,70 | 100 | 2,77 |

Die Verweilzeit k berechnet sich hierbei als Quotient aus IL Volumen durch den Organik-Zulauf. Die Konstante k ist hierbei die berechnete Geschwindigkeitskonstante der Isomerisierung von Methycyclopentan zu Cyclohexan für einen Reaktor mit idealer Rückvermischung inklusive Rückreaktion.

Bei Vergleich der Werte aus Tabelle 2 und 4 lässt sich erkennen, dass ein höheres Volumenverhältnis (Ionische Flüssigkeit/Organik= 4:1) einen höheren Methylcyclopentan-Umsatz hervorruft und somit eine bessere Raum-Zeit-Ausbeute aufweist (11,24 g/h Cyclohexan bei hohem Verhältnis vs. 9,7 g/h Cyclohexan bei niedrigem Verhältnis).

## Patentansprüche

1. Verfahren zur Isomerisierung von Methylcyclopentan (MCP) zu Cyclohexan in Gegenwart einer ionischen Flüssigkeit, **dadurch gekennzeichnet, dass** die Isomerisierung von Methylcyclopentan (MCP) zu Cyclohexan in einer Dispersion (D1) durchgeführt wird, wobei in der Dispersion (D1) die Phase (B) in der Phase (A) dispergiert ist, das Volumenverhältnis der Phase (A) zu Phase (B) im Bereich von 2,5 bis 4 zu 1 [Vol/Vol] liegt, die Phase (A) zu größer 50 Gew.-% mindestens eine ionische Flüssigkeit enthält und die Phase (B) zu größer 50 Gew.-% mindestens einen Kohlenwasserstoff enthält.

2. Verfahren gemäß Anspruch 1, **dadurch gekennzeichnet, dass** die in der Phase (A) enthaltene ionische Flüssigkeit als Kation ein zumindest teilweise alkyliertes Ammoniumion oder ein heterocyclisches Kation und/oder als Anion ein Chloroalumination mit der Zusammensetzung AlₙCl₍₃ₙ₊₁₎ mit 1 <n<2,5 enthält.

3. Verfahren gemäß einem der Ansprüche 1 oder 2, **dadurch gekennzeichnet, dass** die Phase (B) vor der Isomerisierung als Kohlenwasserstoff Methylcyclopentan (MCP) oder ein Gemisch aus Methylcyclopentan (MCP) mit mindestens einem weiteren Kohlenwasserstoff, ausgewählt aus Cyclohexan, n-Hexan, iso-Hexane n-Heptan, iso-Heptane oder Dimethylcyclopentane, enthält.

4. Verfahren gemäß einem der Ansprüche 1 bis 3, **dadurch gekennzeichnet, dass** D1 zusätzlich HCl enthält und/oder gasförmiges HCl in die Dispersion (D1) eingeleitet wird.

5. Verfahren gemäß einem der Ansprüche 1 bis 4, **dadurch gekennzeichnet, dass** die Isomerisierung in einem Rührkessel oder einer Rührkesselkaskade durchgeführt wird.

6. Verfahren gemäß einem der Ansprüche 1 bis 5, **dadurch gekennzeichnet, dass** aus der Vorrichtung, in der die Isomerisierung durchgeführt wird, ein Strom (S1) ausgeleitet wird, der Strom (S1) zumindest eine Teilmenge der Dispersion (D1), in der die Phase (B) in der Phase (A) dispergiert ist, enthält und in der Phase (B) mindestens ein Kohlenwasserstoff enthalten ist, der bei der Isomerisierung hergestellt wurde.

7. Verfahren gemäß Anspruch 6, **dadurch gekennzeichnet, dass** der Strom (S1) in eine Phasentrenneinheit, vorzugsweise in einen Phasenscheider, eingeleitet wird.

8. Verfahren gemäß Anspruch 7, **dadurch gekennzeichnet, dass** in der Phasentrenneinheit ein Strom (S4), enthaltend mindestens 70 Gew.-%, bevorzugt mindestens 90 Gew.-%, an Phase (A), und ein Strom (S5), enthaltend mindestens 70 Gew.-%, bevorzugt mindestens 90 Gew.-%, an Phase (B), voneinander abgetrennt werden.

9. Verfahren gemäß Anspruch 8, **dadurch gekennzeichnet, dass** der Strom (S4) in die Vorrichtung, in der die Isomerisierung, durchgeführt wird, rückgeleitet wird.

10. Verfahren gemäß einem der Ansprüche 6 bis 9, **dadurch gekennzeichnet, dass** in den Strom (S1) ein Strom (S2), enthaltend mindestens 70 Gew.-%, bevorzugt mindestens 90 Gew.-%, an Phase (B), eingeleitet wird, wobei sich ein Strom (S3) ausbildet, enthaltend eine Dispersion (D2), in der die Phase (A) in der Phase (B) dispergiert ist.

11. Verfahren gemäß Anspruch 10, **dadurch gekennzeichnet dass** der Strom (S3) in die Phasentrenneinheit gemäß Anspruch 7 eingeleitet wird.

12. Verfahren gemäß Anspruch 11, **dadurch gekennzeichnet, dass** der Strom (S2) aus dem Strom (S5) gemäß Anspruch 8 abgezweigt und in den Strom (S1) vor oder in die Phasentrenneinheit rückgeleitet wird.

13. Verfahren gemäß einem der Ansprüche 8 bis 12, **dadurch gekennzeichnet, dass** aus dem Strom (S5) Cyclohexan isoliert wird.

## Claims

1. A process for isomerizing of methylcyclopentane (MCP) to cyclohexane in the presence of an ionic liquid, which comprises performing the isomerization of methylcyclopentane (MCP) to cyclohexane in a dispersion (D1), with dispersion of phase (B) in phase (A) in dispersion (D1), the volume ratio of phase (A) to phase (B) being in the range from 2.5 to 4:1 [vol/vol], phase (A) comprising greater than 50% by weight of at least one ionic liquid, and phase (B) comprising greater than 50% by weight of at least one hydrocarbon.

2. The process according to claim 1, wherein the ionic liquid present in phase (A) comprises, as a cation, an at least partly alkylated ammonium ion or a heterocyclic cation and/or, as an anion, a chloroaluminate ion having the composition AlₙCl₍₃ₙ₊₁₎ where 1 < n < 2.5.

3. The process according to either of claims 1 and 2, wherein the hydrocarbon present in phase (B) prior to the isomerization is methylcyclopentane (MCP) or a mixture of methylcyclopentane (MCP) with at least one further hydrocarbon selected from cyclohexane, n-hexane, isohexanes, n-heptane, isoheptanes and dimethylcyclopentanes.

4. The process according to any of claims 1 to 3, wherein D1 additionally comprises HCl and/or gaseous HCl is introduced into dispersion (D1).

5. The process according to any of claims 1 to 4, wherein the isomerization is performed in a stirred tank or a stirred tank cascade.

6. The process according to any of claims 1 to 5, wherein a stream (S1) is discharged from the apparatus in which the isomerization is performed, stream (S1) comprises at least a portion of the dispersion (D1) in which phase (B) is dispersed in phase (A), and phase (B) comprises at least one hydrocarbon which has been prepared in the isomerization.

7. The process according to claim 6, wherein stream (S1) is introduced into a phase separation unit, preferably into a phase separator.

8. The process according to claim 7, wherein, in the phase separation unit, a stream (S4) comprising at least 70% by weight, preferably at least 90% by weight, of phase (A), and a stream (S5) comprising at least 70% by weight, preferably at least 90% by weight, of phase (B), are separated from one another.

9. The process according to claim 8, wherein stream (S4) is recycled into the apparatus in which the isomerization is performed.

10. The process according to any of claims 6 to 9, wherein a stream (S2) comprising at least 70% by weight, preferably at least 90% by weight, of phase (B) is introduced into stream (S1) to form a stream (S3) comprising a dispersion (D2) in which phase (A) is dispersed in phase (B).

11. The process according to claim 10, wherein stream (S3) is introduced into the phase separation unit according to claim 7.

12. The process according to claim 11, wherein stream (S2) is branched off from stream (S5) according to claim 8 and recycled into stream (S1) upstream of or into the phase separation unit.

13. The process according to any of claims 8 to 12, wherein cyclohexane is isolated from stream (S5).

## Revendications

1. Procédé d'isomérisation de méthylcyclopentane (MCP) en cyclohexane en présence d'un liquide ionique, **caractérisé en ce que** l'isomérisation de méthylcyclopentane (MCP) en cyclohexane est réalisée dans une dispersion (D1), la phase (B) étant dispersée dans la phase (A) dans la dispersion (D1), le rapport volumique entre la phase (A) et la phase (B) se situant dans la plage allant de 2,5 à 4 sur 1 [vol/vol], la phase (A) contenant plus de 50 % en poids d'au moins un liquide ionique et la phase (B) contenant plus de 50 % en poids d'au moins un hydrocarbure.

2. Procédé selon la revendication 1, **caractérisé en ce que** le liquide ionique contenu dans la phase (A) contient en tant que cation un ion ammonium au moins partiellement alkylé ou un cation hétérocyclique et/ou en tant qu'anion un ion chloroaluminate de composition AlₙCl₍₃ₙ₊₁₎ avec 1 < n < 2, 5.

3. Procédé selon l'une quelconque des revendications 1 ou 2, **caractérisé en ce que** la phase (B) contient avant l'isomérisation en tant qu'hydrocarbure du méthylcyclopentane (MCP) ou un mélange de méthylcyclopentane (MCP) avec au moins un autre hydrocarbure, choisi parmi le cyclohexane, le n-hexane, les iso-hexanes, le n-heptane, les iso-heptanes ou les diméthylcyclopentanes.

4. Procédé selon l'une quelconque des revendications 1 à 3, **caractérisé en ce que** D1 contient en outre de l'HCl et/ou de l'HCl gazeux est introduit dans la dispersion (D1).

5. Procédé selon l'une quelconque des revendications 1 à 4, **caractérisé en ce que** l'isomérisation est réalisée dans une cuve agitée ou une cascade de cuves agitées.

6. Procédé selon l'une quelconque des revendications 1 à 5, **caractérisé en ce qu'**un courant (S1) est déchargé du dispositif dans lequel l'isomérisation est réalisée, le courant (S1) contenant au moins une partie de la dispersion (D1), dans laquelle la phase (B) est dispersée dans la phase (A), et au moins un hydrocarbure qui a été fabriqué lors de l'isomérisation étant contenu dans la phase (B).

7. Procédé selon la revendication 6, **caractérisé en ce que** le courant (S1) est introduit dans une unité de séparation de phases, de préférence dans un séparateur de phases.

8. Procédé selon la revendication 7, **caractérisé en ce qu'**un courant (S4), contenant au moins 70 % en poids, de préférence au moins 90 % en poids, de phase (A), et un courant (S5), contenant au moins 70 % en poids, de préférence au moins 90 % en poids, de phase (B), sont séparés l'un de l'autre dans l'unité de séparation de phases.

9. Procédé selon la revendication 8, **caractérisé en ce que** le courant (S4) est réintroduit dans le dispositif dans lequel l'isomérisation est réalisée.

10. Procédé selon l'une quelconque des revendications 6 à 9, **caractérisé en ce qu'**un courant (S2), contenant au moins 70 % en poids, de préférence au moins 90 % en poids, de phase (B), est introduit dans le courant (S1), un courant (S3) étant ainsi formé, contenant une dispersion (D2) dans laquelle la phase (A) est dispersée dans la phase (B).

11. Procédé selon la revendication 10, **caractérisé en ce que** le courant (S3) est introduit dans l'unité de séparation de phases selon la revendication 7.

12. Procédé selon la revendication 11, **caractérisé en ce que** le courant (S2) est dérivé du courant (S5) selon la revendication 8 et réintroduit dans le courant (S1) avant ou dans l'unité de séparation de phases.

13. Procédé selon l'une quelconque des revendications 8 à 12, **caractérisé en ce que** du cyclohexane est isolé à partir du courant (S5).
